# EUROPEAN PATENT APPLICATION

(11) **EP 1 316 436 A1**
(43) Date of publication of application: **04.06.2003**
(21) Application number: 01958393.9
(22) Date of filing: 22.08.2001
(51) Int. Cl.: B41M 5/26, G11B 7/24, C07D 487/22, C09B 47/04

(54) **OPTICAL INFORMATION RECORDING MEDIUM**

(30) Priority: 23.08.2000 JP 2000252323
(71) Applicant: Mitsui Chemicals, Inc., Tokyo 100-6070 (JP)
(72) Inventor: NARA, Ryousuke, Sodegaura-shi, Chiba 299-0265 (JP); TANIGUCHI, Yoshiteru, Sodegaura-shi, Chiba 299-0265 (JP); MIHARA, Norihiko, Sodegaura-shi, Chiba 299-0265 (JP); KOIKE, Tadashi, Sodegaura-shi, Chiba 299-0265 (JP); OSUKA, Atsuhiro, Kyoto-shi Kyoto 606-8224 (JP)
(74) Representative: Stuart, Ian Alexander
(86) International application number: JP0107178
(87) International publication number: WO02016144

(57) **Abstract**

An optical information recording medium comprising a transparent substrate and a recording layer formed thereon, wherein the recording layer contains an extended porphyrin compound represented by the following general formula (1): 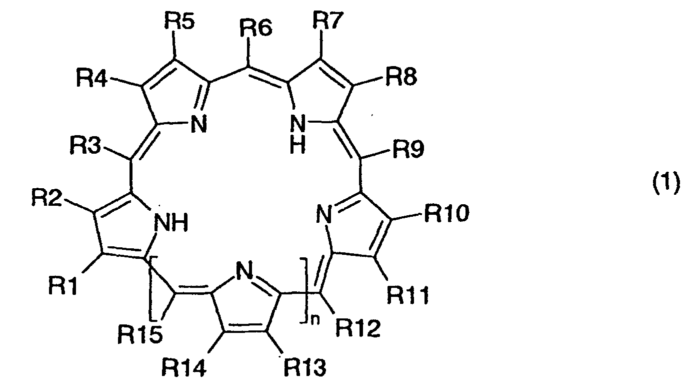 (wherein R₁ to R₁₅ are each a group described in the specification, and n is an integer of 1 or more).

## Description

### Technical Field

The present invention relates to an optical information recording medium, particularly an optical information recording medium of recordable type capable of conducting recording and reproduction using a red and/or blue laser.

### Prior Art

Of recording media conducting recording using a light, a compact disc of recordable type (CD-R) is capable of conducting recording only once and has compatibility with an ordinary CD-ROM; therefore, is being used by many users. Recording media recordable also as a digital versatile disc (DVD) having a higher recording density and capacity than those of CD are being developed; of them, a DVD of recordable type (DVD-R) is expected to spread for its compatibility with DVD-ROM. In these recordable media, an organic dye is used in a recording layer; and the dye, when irradiated with a laser beam, is heated locally at the irradiated portion of the recording layer and causes chemical or physical changes such as decomposition, vaporization, melting-solidification and the like, whereby pits are formed and information recording is made possible.

The organic dye used in the recording layer is required to have a high reflectance before recording so as to give a large modulation factor; therefore, the organic dye is desired to have an absorption peak of large absorption coefficient at about a reproduction wavelength and a high refractive index.

As an organic dye having a high absorption coefficient and a high refractive index, cyanine dyes are mentioned. However, these cyanine dyes are known to generate singlet state oxygen owing to the self-sensitization and easily undergo photo-deterioration. Hence, a metal complex typified by nickel-dithiolate complex has often been mixed, as an oxygen quencher, into the cyanine dye; this has given improved light resistance but has incurred lower recording characteristic and a higher cost. Therefore, the dye used in the recording layer is desired to have by itself both light resistance and a high refractive index.

### Disclosure of the Invention

The present inventors made a study in order to solve the above problems. As a result, the present inventors found out that when an extended porphyrin compound is used in the recording layer of an optical recording medium, the compound shows both good light resistance and a high refractive index. The present invention has been completed based on the above finding.

That is, the present invention lies in the followings.
<1> An optical information recording medium comprising a transparent substrate and a recording layer formed thereon, wherein the recording layer contains an extended porphyrin compound represented by the following general formula (1): 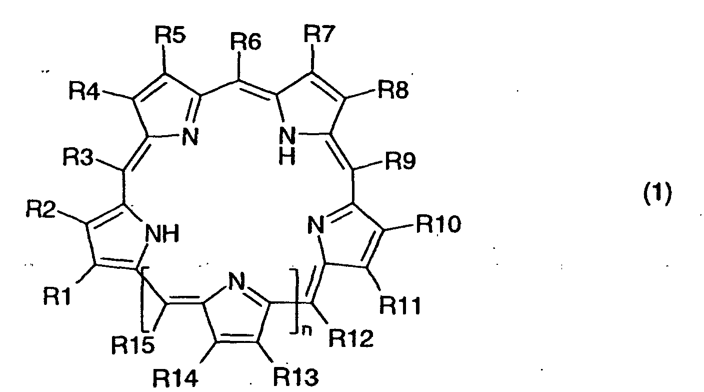 (wherein R₁ to R₁₅ are each independently hydrogen, a halogen, a hydroxyl group, a mercapto group, an amino group, a nitro group, a cyano group, a carboxyl group, a sulfonic acid group, a substituted or unsubstituted alkyl group, a substituted or unsubstituted aryl group, a substituted or unsubstituted alkoxy group, a substituted or unsubstituted aryloxy group, a substituted or unsubstituted alkylthio group, a substituted or unsubstituted arylthio group, a substituted or unsubstituted alkylamino group, a substituted or unsubstituted arylamino group, a substituted or unsubstituted carboxylic acid ester group, a substituted or unsubstituted carboxylic acid amide group, a substituted or unsubstituted sulfonic acid ester group, a substituted or unsubstituted sulfonamide group, a substituted or unsubstituted carbonyl group, a substituted or unsubstituted silyl group or a substituted or unsubstituted siloxy group; n is an integer of 1 to 8; and metal atoms may coordinate to pyrrole nitrogen atoms of the porphyrin ring to form a complex).
<2> The optical information recording medium according to <1>, wherein the extended porphyrin represented by the general formula (1) is in a neutral state or forms, as a bivalent anion, a complex with one or more metals selected from the following metals or with one or more metal compounds thereof:
   Zn, Mg, Ca, Sr, Ba, Sc, Y, La, Ce, Pr, Nd, Sm, Eu, Gd, Tb, Dy, Ho, Er, Tm, Yb, Lu, Ti, Zr, Hf, V, Nb, Ta, Th, U, Cr, Mo, W, Mn, Tc, Re, Fe, Ru, Os, Co, Rh, Ir, Ni, Pd, Pt, Cu, Ag, Au, Cd, Hg, Al, Ga, In, Tl, Si, Ge, Sn, Pb, Sb and Bi.
<3> The optical information recording medium according to <1> or <2>, which has, on a transparent substrate, one or more recording layers, a metal reflecting layer and one or more protective layers and wherein at least one of the recording layers contains an extended porphyrin compound represented by the general formula (1).
<4> The optical information recording medium according to <1> or <2>, which is constituted by a transparent substrate, one or more recording layers formed thereon, and a substrate adhered onto the recording layers via an adhesive layer and wherein at least one of the recording layers contains an extended porphyrin compound represented by the general formula (1).

### Brief Description of the Drawing

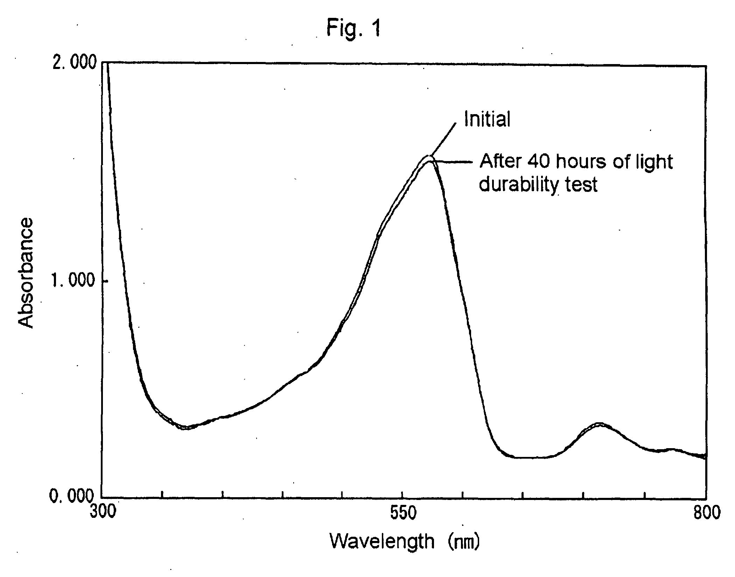

Fig. 1 shows the thin film spectra before and after light resistance test, of the compound shown in Example 1.

### Best Mode for Carrying Out the Invention

In the present recording medium, the heterocyclic rings (pyrrole rings) possessed by the extended porphyrin compound contained in the recording layer, desirably have substituents introduced for improvements in solubility, coatability and durability. However, the heterocyclic rings may not have substituents.

As the substituents other than hydrogen, introduced into R₁ to R₁₅ of the extended porphyrin compound, there are specifically mentioned halogens such as fluorine, chlorine, bromine and iodine; hydroxyl group; mercapto group; nitro group; cyano group; amino group; sulfonic acid group; unsubstituted alkyl groups such as methyl, ethyl, n-propyl, i-propyl, n-butyl, sec-butyl, tert-butyl, n-pentyl, i-pentyl, n-hexyl, n-heptyl, n-octyl, nonyl, decyl, dodecyl and cyclohexyl; substituted alkyl groups such as methylcyclohexyl, ethynyl, propenyl and benzyl; aryl groups such as phenyl, toluyl and xylyl; alkoxy groups such as methoxy, ethoxy, propoxy, butoxy and heptoxy; aryloxy groups such as phenoxy; alkylthio groups such as methylthio, ethylthio, butylthio, heptylthio and hexylthio; alkylamino groups such as (mono or di)methylamino, (mono or di)ethylamino, (mono or di)butylamino and (mono or di)pentylamino; arylamino groups such as (mono or di)phenylamino; carboxylic acid ester groups such as methylcarboxy, ethylcarboxy, butylcarboxy and phenylcarboxy; sulfonic acid (e.g. methylsulfonic acid or benzenesulfonic acid) ester groups; carboxylic acid amide groups such as ethylaminocarboxy and phenylaminocarboxy; sulfonamide groups such as ethylsulfonamide, phenylsulfonamide and benzylsulfonamide; carbonyl groups such as acetyl, ethylcarbonyl, butylcarbonyl and phenylcarbonyl; silyl groups such as trimethylsilyl, triethylsilyl, tripropylsilyl, tributylsilyl and triphenylsilyl; siloxy groups such as trimethoxysilyl; and so forth. The substituents are not restricted thereto. The substituents also include those substituents in which other substituent is added to some of the above-mentioned substituents, for example, halogenated alkyls, halogenated aryls, hydroxyalkyls and hydroxyaryls.

Of these substituents, adjacent substituents, for example, two substituents on a pyrrole ring, or a substituent on a pyrrole ring and a substituent on a methine group connecting two pyrrole rings, may bond to each other to form a new ring.

Such an extended porphyrin compound can be obtained by subjecting a substituted or unsubstituted pyrrole and an appropriate aldehyde to a condensation reaction using, for example, the method described in a literature "M. Neves et al., Chem. Comm., 1999, p. 385". 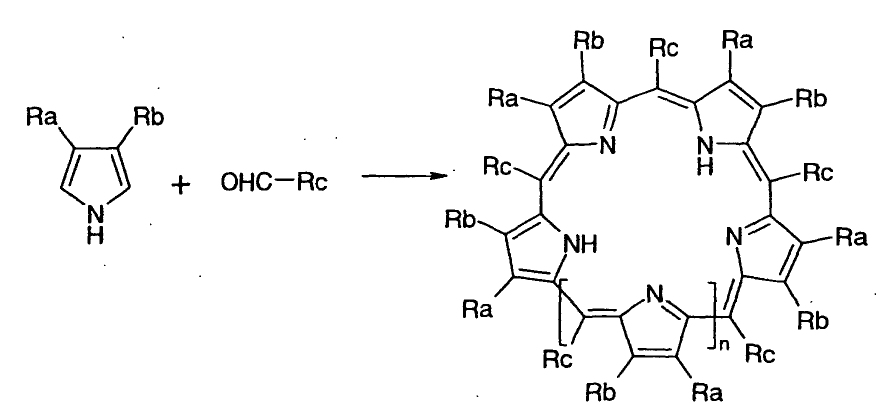 (wherein Ra, Rb and Rc have the same definitions as given for R₁ to R₁₅, and n is an integer of 1 or more).

Then, purification by a cyclic distillation, etc. are conducted for separation from unreacted materials, whereby a mixture of compounds having different n's is obtained. The mixture is further subjected to chromatography or the like, whereby a compound having an intended n can be separated. This compound has a tendency that, with an increase in the n, the absorption wavelength shifts to a longer wavelength; therefore, a compound having a desired wavelength characteristic may be selected and used. A mixture of compounds having different n's may also be used. Incidentally, pentaphyrin of n=1 or hexaphyrin of n=2 is preferred.

In the general formula (1), introduction of different substituents into each pyrrole ring and meso position can be performed by conducting a reaction using a plurality of pyrroles and/or a plurality of aldehydes. Incidentally, the conformation of the extended porphyrin may be any of a state in which each pyrrole nitrogen is directed to the inside of the porphyrin ring and each meso position substituent is directed to the outside of the porphyrin ring as shown in formula (1), and a state in which each pyrrole nitrogen is directed to the outside of the porphyrin ring and each meso position substituent is directed to the inside of the porphyrin ring. Further, the position of NH proton may be at any pyrrole nitrogen of the porphyrin ring owing to conjugation.

The extended porphyrin represented by the general formula (1), used in the present invention may be in a neutral state or may form, as a bivalent anion, a complex with one or more metals or with one or more metal compounds. As the metals, there are mentioned Zn, Mg, Ca, Sr, Ba, Sc, Y, La, Ce, Pr, Nd, Sm, Eu, Gd, Tb, Dy, Ho, Er, Tm, Yb, Lu, Ti, Zr, Hf, V, Nb, Ta, Th, U, Cr, Mo, W, Mn, Tc, Re, Fe, Ru, Os, Co, Rh, Ir, Ni, Pd, Pt, Cu, Ag, Au, Cd, Hg, Al, Ga, In, Tl, Si, Ge, Sn, Pb, Sb, Bi, etc. The metals may each coordinate to one or more pyrrole nitrogens of the extended porphyrin ring; further, other atom (e.g. halogen) or atomic group may coordinate to each metal.

Representative examples of the extended porphyrin according to the present invention are shown below; however, the present compound is not restricted thereto.

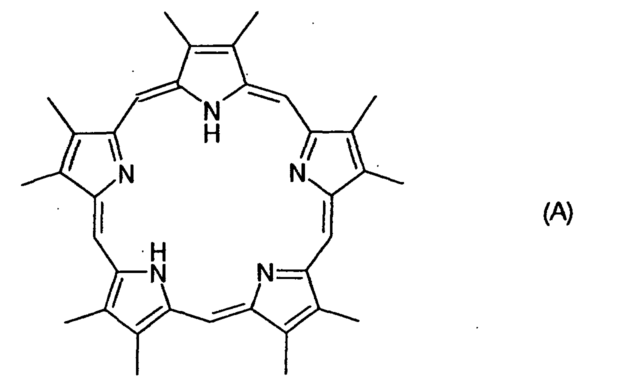

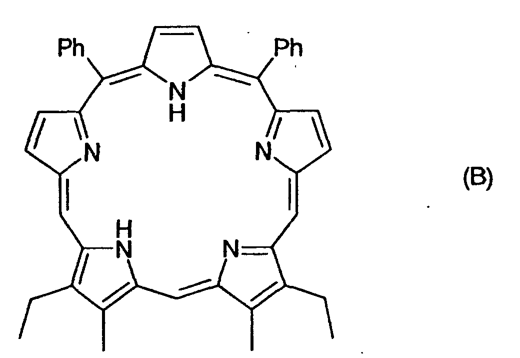

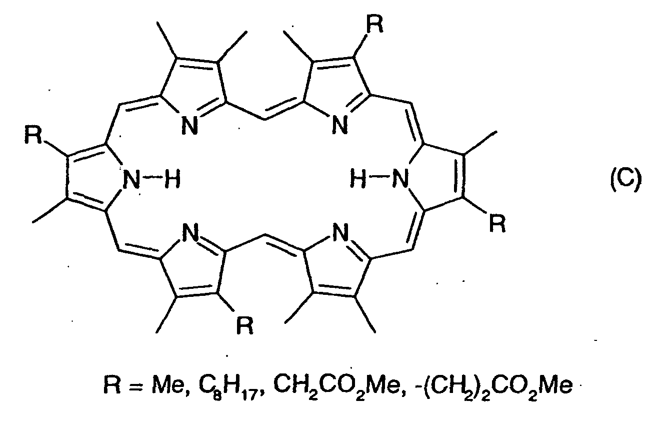

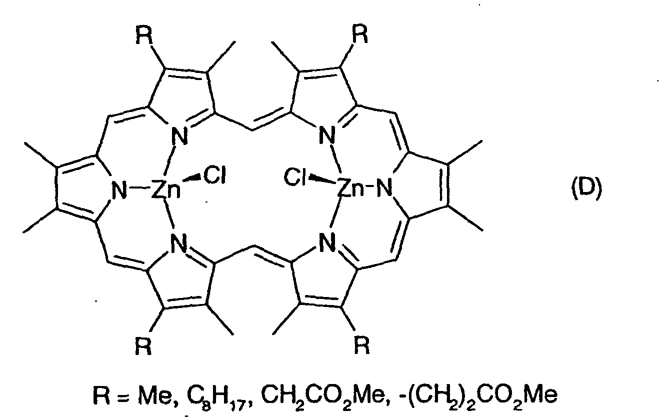

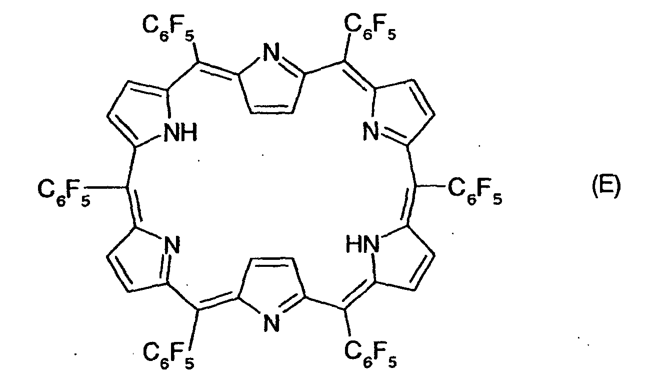

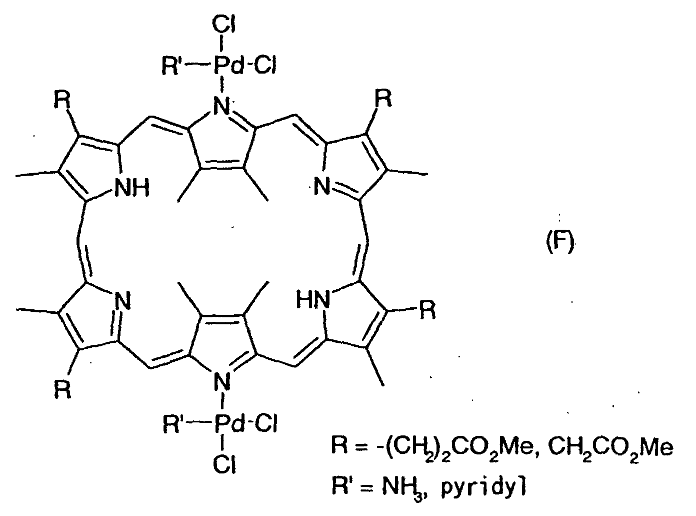

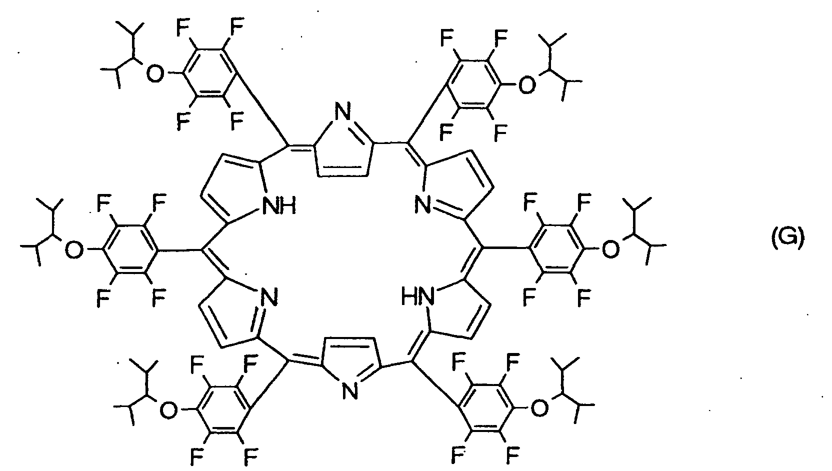

In the optical information recording medium of the present invention, a single-substrate type ordinarily consists of a transparent substrate, a recording layer, a metal reflecting layer and a protective layer, and may further have a foundation layer and a protective layer for improved properties.

The substrate used in the present invention may be any transparent material because recording and reproduction are conducted optically. There can be used, for example, organic polymer materials such as polycarbonate, polyacrylate, polymethacrylate, polystyrene, polyvinyl chloride, polyester, polyolefin, epoxy resin and the like; and inorganic materials such as glass and the like. A polycarbonate resin, which is well balanced between light transmittance and heat resistance and allows easy molding, is preferred particularly. A polyolefin having a cyclic skeleton is also desirable because it has low anisotropy and shows low water absorption.

The substrate may have, on the surface, guide grooves or pits indicating recording positions, or some of the pits for information, etc. exclusively used for reproduction. These grooves, pits, etc. are ordinarily formed at the time of substrate production conducted by injection molding or casting, but may be formed by a laser cutting method or a photo-polymer method.

The recording layer of the optical information recording medium of the present invention contains a compound represented by the general formula (1), and can conduct recording by applying a laser beam thereto to decompose the compound to form pits. In the recording layer, one or more kinds of other dyes may be mixed into the dye represented by the general formula (1).

Further, light-non-absorbing substances may be added to the dye represented by the general formula (1) or its mixture with other dye(s) for improved recording characteristic and durability.

Specific examples of the dyes other than the dye represented by the general formula (1), used in the recording layer are large ring azaanulene dyes (for example, phthalocyanine dyes, naphthalocyanine dyes and azaporphyrin dyes having 1 to 4 meso-position nitrogen atoms); polymethine dyes (for example, cyanine dyes, merocyanine dyes, and squarylium dyes); anthraquinone dyes; azulenium dyes; azo dyes; and indoaniline dyes. Of these dyes, phthalocyanine dyes having high durability and light resistance are desired particularly.

The dye-containing recording layer may be a laminated layer consisting of two or more layers. In this case, the dye represented by the general formula (1) may be used only in one layer or in two or more layers.

It is also possible to form the recording layer in a laminated structure consisting of two or more layers and allows one of the layers to contain a dye represented by the general formula (1) and other layer(s) to contain a dye(s) other than the dye represented by the general formula (1) so as to allow for multi-wavelength recording. As the dye(s) used in the recording layer(s) other than the layer containing the dye represented by the general formula (1), there are mentioned large ring azaanulene dyes (for example, phthalocyanine dyes, naphthalocyanine dyes and azaporphyrin dyes having 1 to 4 meso-position nitrogen atoms); polymethine dyes (for example, cyanine dyes, merocyanine dyes, and squarylium dyes); anthraquinone dyes; azulenium dyes; azo dyes; indoaniline dyes; and so forth. However, a dye(s) different in optical characteristic from the dye of the general formula (1) is (are) selected.

The recording layer containing such a dye(s) can be formed ordinarily by spin coating, spray coating, dip coating, roll coating, etc. In the coating, substances constituting the recording layer, such as dye, resinous binder and the like are dissolved in a solvent giving no damage to the substrate used, to prepare a coating solution; and the solution is coated on the substrate and dried to form a recording layer. As the solvent, there are preferably used aliphatic or alicyclic hydrocarbons such as hexane, heptane, octane, decane and cyclohexane; aromatic hydrocarbons such as toluene and xylene; ethers such as diethyl ether, dibutyl ether and tetrahydrofuran; alcohols such as methanol, ethanol, isopropanol, tetrafluoropropanol and methyl cellosolve; halogen compounds such as chloroform, dichloromethane and 1,2-dichloroethane; and so forth. These solvents may be used singly or in admixture of two or more kinds. When the recording layer is formed in a laminated structure consisting of two or more layers, it is desired to use a solvent which does not adversely affect the layer formed earlier.

The recording layer may also be formed by vacuum deposition. This method is effective when the substances to constitute the recording layer have low solubility in solvents or when it is impossible to select a solvent which gives no damage to the substrate used.

The recording layer has a thickness of 10 to 300 nm, preferably 50 to 200 nm.

A foundation layer may be formed between the substrate and the recording layer for the purposes of, for example, prevention of recording layer from deterioration. It is possible to use, for example, a layer composed of an organic material (e.g. a polystyrene or a polymethacrylate) or an inorganic material (e.g. SiO₂). These materials may be used singly or in admixture of two or more kinds of organic materials or two or more kinds of inorganic materials. Or, two or more kinds of these materials may be used in laminated layers; that is, laminated layers each containing a different organic material, laminated layers each containing a different inorganic material, or laminated layers consisting of a layer containing an organic material and a layer containing an inorganic material may be formed.

On the recording layer may be formed a reflecting layer using a metal such as Au, Al, Pt, Ag and Ni, or an alloy thereof. In particular, Au is desirable because it is stable to oxygen and water. Such a reflecting layer is formed by vapor deposition, sputtering, ion plating, etc. The reflecting layer has a thickness of 10 to 300 nm, desirably 30 to 150 nm. Incidentally, the reflecting layer is formed at a side opposite to the side of light incidence; therefore, when light incidence is made from the protective layer or dummy substrate (both described later) side, the reflecting layer is formed at the substrate side relative to the recording layer.

Between the recording layer and the reflecting layer, an intermediate layer may be formed in order to achieve, for example, an increased adhesivity or an increased reflectance.

A protective layer may be formed on the reflecting layer or the recording layer. There is no particular restriction as to the protective layer as long as the layer protects the reflecting layer or the recording layer from an external force. There are ordinarily mentioned polymer materials such as acrylate or methacrylate polymer obtained by general radical polymerization or epoxy polymer obtained by photo-induced cationic polymerization. These polymer materials may be obtained by homopolymerization or copolymerization with other monomer, oligomer or the like. These materials may also be diluted with a solvent and coated. Among them, ultraviolet curing resins, for example, acrylate resins such as urethane acrylate, epoxy acrylate and polyester acrylate are desired for the workability.

The protective layer is formed by spin coating, dip coating, bar coating, screen printing, etc. Spin coating is employed in many cases for the workability.

The protective layer has a thickness of generally 1 to 100 µm. In the present invention, a thickness of 1 to 20 µm is preferred.

The thus-formed protective layer protects the recording layer or the reflecting layer. When a strong protective layer is required depending upon the use condition of the resulting recording medium, it is possible to form an inorganic or organic protective layer on the protective layer made of the above-mentioned polymer material. As the inorganic protective layer, there are mentioned SiO₂, Si₃N₄, MgF₂, AlN, SnO₂, etc. As the organic protective layer, there are mentioned a thermosetting resin, an electron beam curing resin, an ultraviolet curing resin, etc. When an organic protective layer is formed using a thermosetting resin, the thermosetting resin is dissolved in an appropriate solvent to prepare a coating fluid, followed by coating of the fluid and subsequent drying and curing. When an organic protective layer is formed using an ultraviolet-curing resin, a coating fluid is prepared from the ultraviolet-curing resin per se or by dissolving the resin in an appropriate solvent, the coating fluid is coated, and an ultraviolet light is applied to cure the resin. An inorganic protective layer can be formed by vapor deposition, etc. These materials may be used singly or in admixture. The protective layer may be formed not only in a single layer but also in a plurality of layers for increased adhesivity with other layer and other purpose.

In other constitution of the present recording medium, a substrate having a recording layer formed thereon may be laminated with another substrate. In this case, the another substrate may be a dummy substrate having no layer (e.g. recording layer) formed thereon; or, a substrate having a recording layer formed thereon may be laminated with another substrate having a recording layer, a reflecting layer, etc. formed thereon, in such a state that the sides of the two substrates having respective recording layers face each other. As the adhesion method employed in this case, there are mentioned, for example, a hot melt method, a method using an ultraviolet curing adhesive, and a method using a one-pack or two-pack type reactive adhesive.

### Examples

The present invention is specifically described below with reference to Examples. However, the present invention is in no way restricted to these Examples.

### Example 1

Synthesis method and characteristic values are shown below on a representative compound of the present invention, i.e. meso-hexa(pentafluorophenyl)hexaphyrin which is a n=2 derivative of formula (1) [the illustrative compound (E) shown previously].

### [Synthesis method]

Pyrrole was dropwise added to pentafluorobenzaldehyde which was being refluxed in glacial acetic acid-nitrobenzene. The resulting mixture was refluxed for a further 45 minutes and then subjected to distillation to remove the solvent. The residue was purified using a silica-filled column to obtain an intended product.

### [UV spectrum of film]

The above-obtained hexaphyrin of formula (E) was dissolved in chloroform so as to give a concentration of 10 g/l. The resulting solution was coated on a glass chip by spin coating, to form a film of 120 nm in thickness. The thin film was measured for UV spectrum. The UV spectrum is shown in Fig. 1.

### [Measurement of optical constants]

The hexaphyrin of formula (E) was vapor-deposited on a glass substrate-in 8 different thicknesses between 45 nm and 120 nm, to prepare samples. Each sample was measured for absorption spectrum and reflection spectrum, from which refractive indexes and extinction coefficients at various wavelengths were calculated.

A balance between high refractive index and proper extinction coefficient was obtained in a wavelength range of 630 to 670 nm which is used in ordinary DVD's. The results are shown in the following Table 1.

| Wavelength | Refractive index | Extinction coefficient |
|---|---|---|
| 630 nm | 2.45 | 0.28 |
| 640 nm | 2.29 | 0.17 |
| 650 nm | 2.09 | 0.13 |
| 660 nm | 2.02 | 0.13 |
| 670 nm | 1.97 | 0.14 |

### [Light durability test]

The dye of formula (E) was coated on a polycarbonate substrate by spin coating, then exposed to a light from a carbon arc, and measured for UV spectrum. A ratio of λmax absorbance after light exposure to λmax absorbance before light exposure was determined and taken as retention. A high retention of 99% was obtained after 40 hours of a light exposure test. In Fig. 1 is shown the thin-film spectrum after the light durability test, together with that before the test.

### [Production of medium]

A film (thickness: about 80 nm) of the dye of formula (E) was formed, by vapor deposition, on a polycarbonate substrate of 0.6 mm in thickness and 120 mm in diameter (pitch between grooves: 0.74 µm, groove width: 0.3 µm, groove depth: 100 nm). Thereon was formed an Au layer in a thickness of 80 nm by sputtering, and further SD-17 of UV-curing type was laminated. Thereon was laminated a dummy substrate of 0.6 mm in thickness, made of a polycarbonate, whereby a trial optical recording medium was produced.

Recording was made for the optical recording medium based on the method specified for DVD, using a DDU tester, a product of Pulstec Industrial Co., Ltd. A good recording characteristic of jitter = 8% was confirmed at a recording power of 10 mW.

### Example 2

A dye of the formula (G) was dissolved in cyclohexane so as to give a concentration of 10 g/l. The resulting solution was coated, by spin coating, on a polycarbonate substrate of 0.6 mm in thickness and 120 mm in diameter (pitch between grooves: 0.74 m, groove width: 0.3 µm, groove depth: 100 nm) to form a film of about 60 nm in thickness. Thereon was formed an Au reflecting layer in a thickness of 80 nm by sputtering, and further SD-17 of UV-curing type was laminated. Thereon was laminated a dummy substrate of 0.6 mm in thickness, made of a polycarbonate, whereby a trial optical recording medium was produced.

Recording was made for the optical recording medium based on the method specified for DVD, using a DDU tester, a product of Pulstec Industrial Co., Ltd. A recording characteristic of jitter = 12% was confirmed at a recording power of 20 mW.

### Industrial Applicability

The extended porphyrin of the general formula (1) used in the present invention as a recording layer dye has by itself durability against light, a high refractive index and an appropriate extinction coefficient; therefore, the present invention can provide an optical information recording medium having good recording characteristic and excellent light durability.

## Claims

1. An optical information recording medium comprising a transparent substrate and a recording layer formed thereon, wherein the recording layer contains an extended porphyrin compound represented by the following general formula (1): 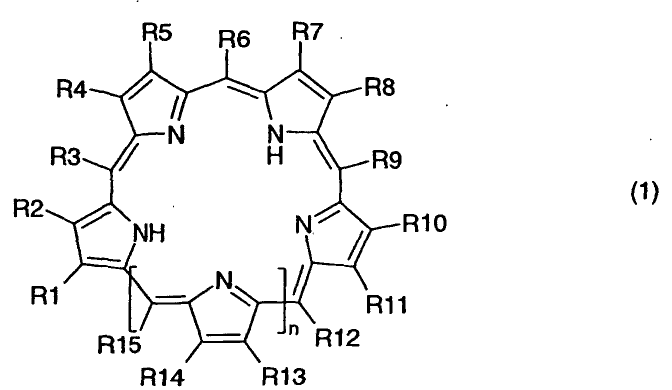 (wherein R₁ to R₁₅ are each independently hydrogen, a halogen, a hydroxyl group, a mercapto group, an amino group, a nitro group, a cyano group, a carboxyl group, a sulfonic acid group, a substituted or unsubstituted alkyl group, a substituted or unsubstituted aryl group, a substituted or unsubstituted alkoxy group, a substituted or unsubstituted aryloxy group, a substituted or unsubstituted alkylthio group, a substituted or unsubstituted arylthio group, a substituted or unsubstituted alkylamino group, a substituted or unsubstituted arylamino group, a substituted or unsubstituted carboxylic acid ester group, a substituted or unsubstituted carboxylic acid amide group, a substituted or unsubstituted sulfonic acid ester group, a substituted or unsubstituted sulfonamide group, a substituted or unsubstituted carbonyl group, a substituted or unsubstituted silyl group or a substituted or unsubstituted siloxy group; n is an integer of 1 to 8; and metal atoms may coordinate to pyrrole nitrogen atoms of the porphyrin ring to form a complex).

2. The optical information recording medium according to Claim 1, wherein the extended porphyrin represented by the general formula (1) is in a neutral state or forms, as a bivalent anion, a complex with one or more metals selected from the following metals or with one or more metal compounds thereof:
Zn, Mg, Ca, Sr, Ba, Sc, Y, La, Ce, Pr, Nd, Sm, Eu, Gd, Tb, Dy, Ho, Er, Tm, Yb, Lu, Ti, Zr, Hf, V, Nb, Ta, Th, U, Cr, Mo, W, Mn, Tc, Re, Fe, Ru, Os, Co, Rh, Ir, Ni, Pd, Pt, Cu, Ag, Au, Cd, Hg, Al, Ga, In, Tl, Si, Ge, Sn, Pb, Sb and Bi.

3. The optical information recording medium according to Claim 1 or 2, which has, on a transparent substrate, one or more recording layers, a metal reflecting layer and one or more protective layers and wherein at least one of the recording layers contains an extended porphyrin compound represented by the general formula (1).

4. The optical information recording medium according to Claim 1 or 2, which is constituted by a transparent substrate, one or more recording layers formed thereon, and a substrate adhered onto the recording layers via an adhesive layer and wherein at least one of the recording layers contains an extended porphyrin compound represented by the general formula (1).
